Europäisches Patentamt

European Patent Office

Office européen des brevets

(19)

(11) Publication number: **0 039 253**
**B1**

## EUROPEAN PATENT SPECIFICATION

(12)

(45) Date of publication of patent specification: **22.06.83**

(21) Application number: **81301918.9**

(22) Date of filing: **30.04.81**

(51) Int. Cl.³: **C 07 C 69/753,**
**C 07 C 67/38**

(54) Process for preparing carboxylic acid esters of dicyclopentadienes.

(30) Priority: **30.04.80 JP 57672/80**

(43) Date of publication of application:
**04.11.81 Bulletin 81/44**

(45) Publication of the grant of the patent:
**22.06.83 Bulletin 83/25**

(84) Designated Contracting States:
**DE FR GB IT NL**

(56) References cited:
**None**

(73) Proprietor: **JAPAN SYNTHETIC RUBBER CO., LTD.**
**11-24, Tsukiji-2-chome Chuo-ku**
**Tokyo (JP)**

(72) Inventor: **Ishikawa, Katuhiro**
**13-13, Aobacho, Ohbaneen Komonocho**
**Mie-gun, Mie-ken (JP)**
Inventor: **Ohno, Ryotaro**
**1-1, Toyotamakami-2-chome**
**Nerima-ku, Tokyo (JP)**
Inventor: **Arakawa, Masatoshi**
**1, Morigayamacho**
**Yokkiachi-shi (JP)**

(74) Representative: **Tubby, David George et al,**
**MARKS & CLERK 57-60 Lincoln's Inn Fields**
**London WC2A 3LS (GB)**

# Process for preparing carboxylic acid esters of dicyclopentadienes

The present invention relates to a process for preparing mono- and di- carboxylate esters of dicyclopentadienes.

Mono- and di- carboxylate esters of dicyclopentadienes are compounds in which one or two esterified carboxy groups has or have been substituted on the basic dicyclopentadiene ring system.

Various processes for preparing such dicyclopentadiene esters are known. For example, one such process comprises first synthesizing a mono- or di- aldehyde or mono- or di- hydroxmethyl derivative of a dicyclopentadiene by an oxo reaction and then oxidizing this intermediate and esterifying the product of the oxidation. However, this method has the disadvantages that the procedure is complicated and the yield is low. In the case of olefins, a hydroesterification reaction of the type required to introduce a carboxylate ester group into a dicyclopentadiene has been developed on the basis of studies of the oxo reaction, in which the olefin is reacted with carbon monoxide and an alcohol. Thus, when and olefin is to be esterified, the olefin, carbon monoxide and the alcohol are reacted in the presence of a catalyst, such as a cobalt carbonyl or a palladium complex. However, since this kind of reaction is usually conducted at a high temperature, application of this technique to dicyclopentadienes has the disadvantage that side reactions, due to pyrolysis of the dicyclopentadiene, take place, resulting in a low yield.

We have now discovered a way of applying this hydroesterification reaction using carbon monoxide and an alcohol in the presence of a cobalt catalyst, hitherto known for olefins, to dicyclopentadienes, to prepare mono- and dicarboxylate esters of the dicyclopentadiene. Specifically, it has been discovered that:

1. A side reaction characteristic of the double bond in the norbornene ring of dicyclopentadienes can be suppressed by adding a certain amount of a pyridine base to the dicyclopentadiene reaction system;

2. by carrying out the hydroesterification of the dicyclopentadiene at a relatively low temperature, specifically 130°C or below, various side reactions arising from pyrolysis of the dicyclopentadiene can be prevented and a monocarboxylate of the dicyclopentadiene can be obtained in a high yield;

3. by subjecting the monocarboxylate thus obtained to a second hydroesterification, it is possible to obtain a dicarboxylate of the dicyclopentadiene in a high yield;

4. in the case referred to in paragraph 3, the reaction rate can be increased by carrying out this second hydroesterification at a relatively high temperature, e.g. at least 130°C; and

5. by maintaining a carbon monoxide pressure of 19.6 bars.G (20 kg/cm².G) or higher, during the preparation of the monocarboxylate the side reaction characteristic of the double bond of the norbornene ring is still more effectively suppressed, thus enabling the mono- or di- carboxylate ester of the dicyclopentadiene to be obtained in a still higher yield.

Thus, the present invention consists in a process for preparing a mono- or di- carboxylate ester of a dicyclopentadiene by reacting the dicyclopentadiene with carbon monoxide and an alcohol in the presence of a cobalt catalyst, characterized in that the hydroesterification reaction (step 1) is effected at a temperature of 130°C or less in the presence of at least 0.5 mole (per mole of the dicyclopentadiene) of a pyridine base to hydroesterify the double bond of the norbornene ring, thus giving the monocarboxylate ester, and optionally subsequently hydroesterifying (step 2) the remaining double bond of said ester to give the dicarboxylate ester of the dicyclopentadiene. The temperature of the hydroesterification reaction is step 2 is preferably, but not necessarily, 130°C or higher.

The term "pyridine base", as used herein, is the collective name for pyridine and its homologues and similar derivatives of pyridine, including methyl derivatives (such as $\beta$-picoline or $\gamma$-picoline), ethyl derivatives (such as 4-ethylpyridine), dimethyl derivatives (such as 3,4-lutidine or 3,5-lutidine), trimethyl and methylethyl derivatives (such as $\beta$-collidine), fused ring derivatives (such as isoquinoline) and similar derivatives of pyridine. In particular, pyridine itself and the picolines are preferred for use as the pyridine base. The pyridine base helps suppress the various aforementioned side reactions and, at the same time, acts as a catalyst for the hydroesterification in the form of a complex with the cobalt compound.

In order to suppress these side reactions, the pyridine base should be added in an amount of 0.5 mole or more, preferably 0.8 mole or more, per mole of the dicyclopentadiene. If the amount is less than 0.5 mole, it is difficult to suppress the side reactions characteristic of the double bond in the norbornene ring and consequently the yield of mono- or di- carboxylate of the dicyclopentadiene is reduced. However, this minimum quantity of pyridine is only necessary in the case of the first hydroesterification reaction (namely the preparation of the monocarboxylate) and the amount of pyridine base present is not critical in the course of the second hydroesterification reaction (namely the preparation of the dicarboxylate). On the other hand, there is no particular upper limit on the amount of the pyridine base, but the use of an excessively large amount of pyridine base may lead to a reduction in the reaction rate and has no practical significance. Usually, therefore, we prefer to use no more than 2 moles of pyridine base per mole of the dicyclopentadiene.

The reaction conditions, which are the second characteristic of the present invention require that the first hydroesterification reaction be carried out at a relatively low temperature. This first hydroesterification reaction involves mainly the hydroesterification of the double bond of the norbornene ring and is carried out at a temperature not greater than 30°C, preferably a temperature from 100°C to 120°C. If this first hydroesterification reaction is carried out at a temperature greater than 130°C, side reactions arising from pyrolysis of the dicyclopentadiene take place and the desired monocarboxylate cannot be obtained in a high yield. The temperature conditions for the second, and optional, hydroesterification reaction, in which the dicarboxylate is prepared by hydroesterification of the remaining double bond are less critical and this reaction is preferably carried out at a temperature in the range from 100 to 160°C, more preferably from 120 to 160°C and most preferably from 130 to 150°C. In particular the reaction rate can be increased by elevating the reaction temperature in the second hydroesterification reaction to 130°C or higher.

When preparing a dicarboxylate, the first hydroesterification and the second hydroesterification may be carried out in succession without any intermediate isolation of the product of the first hydroesterification reaction; in this case, the first hydroesterification reaction may be carried out as described above and then the second hydroesterification reaction may be effected simply by elevating the temperature, preferably to a value more than 130°C. As a modification of this method, it is possible to remove unreacted dicyclopentadiene from the reaction system after the first hydroesterification reaction and then proceed to the second hydroesterification reaction. Alternatively, the second hydroesterification reaction may be carried out discontinuously by separating the monocarboxylate of the dicyclopentadiene from the reaction system prepared by the first hydroesterification reaction and then subjecting this monocarboxylate to the second hydroesterification reaction.

The first hydroesterification and second hydroesterification may be carried out either at a predetermined temperature within the appropriate range, as described above, or by elevating the temperature continuously. If the second hydroesterification reaction is effected by elevating the temperature immediately after the first hydroesterification reaction has been completed, it is preferable that 80% or more of the norbornene ring double bonds should be reacted in the first hydroesterification step. On the other hand, if the first hydroesterification and second hydroesterification are not carried out continuously, it is not necessary that the first hydroesterification reaction should be continued until 80% or more of the norbornene ring double bonds have been reacted, since unreacted dicyclopentadiene is preferably removed after the first hydroesterification.

Of the reaction conditions, the carbon monoxide pressure during the reaction is not critical. However, we prefer that the carbon monoxide pressure should be 19.6 bars.G (20 kg/cm$^2$.G) or more, since this improves catalyst stability and yield. Particularly during the first hydroesterification reaction, namely the preparation of the monocarboxylate, the carbon monoxide pressure is preferably 19.6 bars.G (20 kg/cm$^2$.G) or more, more preferably 49.0 bars.G (50 kg/cm$^2$.G) or more, in order to suppress the side reactions characteristic of the norbornene ring double bond. Increasing the carbon monoxide pressure helps suppress these side reactions and the suppressive effect rapidly increases when the pressure exceeds 19.6 bars.G (20 kg/cm$^2$.G). However, increasing the pressure much more above 49.0 bars.G (50 kg/cm$^2$.G), e.g. to 196 bars.G (200 kg/cm$^2$.G), brings no further advantage.

The carbon monoxide fed to the reaction system preferably has as low as possible a hydrogen content, because carbon monoxide having a high hydrogen content (for example water gas) promotes the formation of an aldehyde by-product, which lowers the yield.

The dicyclopentadiene chosen as starting material will, of course, depend upon the particular mono- or di- carboxylate ester which it is desired to achieve; dicyclopentadiene itself or an alkyl- substituted dicyclopentadiene such as dimethyldicyclopentadiene will normally be used.

The alcohol employed as the other starting material will likewise depend upon the particular ester which it is desired to prepare. Suitable alcohols include such monohydric alcohols as methanol, ethanol or propanol and such polyhydric alcohols as ethylene glycol, propylene glycol, glycerin and pentaerythritol. Although the ratio of the dicyclopentadiene to the alcohol may vary over a wide range, we generally prefer to use from 1.0 to 3.0 moles of alcohol when preparing the mono-carboxylate and from 1.5 to 5.0 moles of alcohol when preparing the dicarboxylate, respectively, per mole of the dicyclopentadiene. When preparing the dicarboxylate, the alcohol may be added to the reaction system either all at once at the beginning of the reaction or in two portions, one at the beginning of the reaction and one after completion of the first hydroesterification. However, where the alcohol is added in two portions, the amount of alcohol added at the beginning is preferably at least 1.0 mole per mole of the dicyclopentadiene.

A variety of compounds may be used as the cobalt catalyst in the process of the invention, but, in order to act most effectively as catalyst in combination with the pyridine base, we prefer to use a cobalt carbonyl or a substance capable of forming a cobalt carbonyl, for example cobalt oxide, cobalt carbonate, cobalt hydroxide, metallic cobalt, cobalt acetate, cobalt naphthenate or cobalt octenate. If a cobalt compound capable of forming a cobalt carbonyl is used, we prefer to employ a preliminary step to convert it to a cobalt carbonyl.

Although the ratio of pyridine base to cobalt catalyst is not critical, we prefer to use from 1.5 to 4.0 moles of the pyridine base per gram atom of cobalt in the cobalt catalyst, since such a combination enables the catalyst to be readily separated from the reaction product after completion of the reaction.

The reaction is preferably effected in the presence of a solvent, the nature of which is not critical, provided that it has no adverse effect upon the reaction. Examples of suitable solvents include: hydrocarbons, such as pentane, hexane, heptane, cyclohexane, benzene, toluene and xylene; and polar compounds, such as acetone, tetrahydrofuran, methyl ethyl ketone and methyol acetate. Although the amount of solvent is not critical, it is preferably employed in an amount from 0 to 2 times the volume of the dicyclopentadiene.

When the reaction is complete, the catalyst can readily be separated by adding to the reaction mixture a hydrocarbon (such as pentane, hexane, heptane, cyclohexane, benzene, toluene or xylene). The catalyst thus separated can be recycled to the reaction system for reuse without any particular treatment or after activation. Operations such as this separation should be effected under an inert atmosphere (for example carbon monoxide or nitrogen) in order to prevent decomposition of the catalyst.

By following the preferred procedures described above, it is possible to obtain the mono- or di- carboxylate ester of the chosen dicyclopentadiene in a high yield and, moreover, to separate and reuse the catalyst after completion of the reaction; thus, it is anticipated that the process of the invention will be particularly advantageous for industrial use.

The mono- and di- carboxylate esters of dicyclopentadienes obtained by the process of the invention can be used as the basic acid component of an unsaturated polyester resin in preparing a variety of materials, particularly for use as coating materials (for example unsaturated polyester resin coating materials, alkyd resin coating materials or oil-free alkyd resin coated materials), and, when so used, have the advantage that they can give rise to remarkable improvements in the boiling water resistance, chemical resistance, water resistance, flexibility, impact resistance, heat resistance, weather resistance and air-dryability, compared with similar resins containing such basic acid components as phthalic anhydride, isophthalic acid, terephthalic acid or fatty acids. Moreover, these dicyclopentadiene carboxylates are cycloaliphatic carboxylates and are thus of great industrial value for use as modifiers for saturated polyesters, plasticizers or lubricants.

The invention is further illustrated by the following non-limiting Examples.

Example 1
Preparation of monocarboxylate

Into a 5 litre autoclave were charged 6 moles of dicyclopentadiene, 6.6 moles of methanol, 7.5 moles of pyridine and 1.5 moles of dicobalt octacarbonyl; carbon monoxide was then introduced into the autoclave to a pressure of 49.0 bars.G ( 50 kg/cm$^2$.G), after which the reaction mixture was heated to 110°C to effect the reaction. Throughout the reaction, the carbon monoxide pressure was maintained at 49.0 bars.G (50 kg/cm$^2$.G), by supplying sufficient carbon monoxide to compensate for that consumed by the reaction; hydroesterification of the double bond of the norbornene ring was thus carried out under these conditions for 3 hours.

At the end of this time, the reaction mixture was cooled, carbon monoxide was removed and the reaction mixture was treated with 3N aqueous hydrochloric acid to decompose the catalyst. The mixture was then washed with water and distilled under reduced pressure, to give 80 g of a fraction boiling at 30—40°C/1 mm Hg (dicyclopentadiene) and 945 g of a fraction boiling at 80—90°C/0.0013 bar (1 mm Hg) (methyl tricyclodecenemonocarboxylate). The saponification value of this fraction boiling at 80—90°C/0.0013 bar (1 mm Hg) was 290, which agreed with the saponification value of methyl tricyclodecenemonocarboxylate, 292. The product was also confirmed to be methyl tricyclodecenemonocarboxylate by GPC (gel permeation chromatography), NMR and IR.

From the results of this distillation, it was calculated that the conversion of dicyclopentadiene was 90 mole % and the yield of methyl tricyclodecenecarboxylate was 82 mole %. The methyl tricyclodecenemonocarboxylate was a transparent, colourless liquid having a viscosity of 14 centipoises at 20°C.

Example 2

The procedure described in Example 1 was repeated, except that the carbon monoxide pressure was 29.4 bar.G (30 kg/cm$^2$.G) instead of 49.0 bar.G (50 kg/cm$^2$.G). It was found from the results of distillation that the conversion of dicyclopentadiene was 91 mole % and the yield of methyl tricyclodecenemonocarboxylate was 76 mole %.

Example 3

The procedure described in Example 1 was repeated, except that 4.8 moles of pyridine, rather than 7.5 moles, were employed. It was found from the results of distillation that the conversion of dicyclopentadiene was 97 mole % and the yield of methyl tricyclodecenemonocarboxylate was 75 mole %.

## Comparative Example 1

The procedure described in Example 1 was repeated, except that the reaction temperature was 135°C and the reaction time was 1 hour. It was found from the results of distillation that the conversion of dicyclopentadiene was 98 mole % and the yield of methyl tricyclodecenemonocarboxylate was 48 mole %.

## Comparative Example 2

The procedure described in Example 1 was repeated, except that the amount of pyridine was reduced from 7.5 moles to 2.5 moles and the amount of dicobalt octacarbonyl was reduced from 1.5 moles to 0.5 mole. It was found from the results of distillation that the conversion of dicyclopentadiene was 83 mole % and the yield of methyl tricyclodecenemonocarboxylate was 38 mole %.

## Example 4
### Preparation of dicarboxylate

Into a 5 litre autoclave were charged 6 moles of dicyclopentadiene, 15 moles of methanol, 7.5 moles of pyridine and 1.5 moles of dicobalt octacarbonyl; carbon monoxide was then introduced into the autoclave to a pressure of 49 bars.G (50 kg/cm².G), and the reaction mixture was heated to 110°C to effect the reaction. The carbon monoxide pressure was maintained at 29.4 bar.G (30 kg/cm².G) by supplying to the autoclave throughout the reaction an amount of carbon monoxide corresponding to that consumed by the reaction. The reaction was carried out for 3 hours under these conditions until the hydroesterification conversion of the norbornene ring double bond had reacted 90%. The reaction temperature was then raised to 140°C and the reaction was continues at this temperature for a further 3 hours.

After cooling the reaction mixture, the carbon monoxide was removed and then the mixture was treated with 3N aqueous hydrochloric acid to decompose the catalyst. The mixture was then washed with water and the solvent was distilled off under reduced pressure, giving 138 g of a fraction boiling at 80—90°C/0.0031 bar (1 mm Hg) (methyl tricyclodecenemonocarboxylate) and 1,224 g of a fraction boiling at 135—140°C/0.0013 bar (1 mm Hg) (dimethyl tricyclodecanedicarboxylate). This latter fraction boiling at 135—140°C/0.013 bar (1 mm Hg), has a saponification value of 443, which agreed with the known value of dimethyl tricyclodecanedicarboxylate (444). The identity of the compound was also confirmed by GPC, NMR and IR.

From the fractions obtained by the above distillation, it was found that the conversion of dicyclopentadiene was 100 mole %, the yield of methyl tricyclodecenetricyclodecanedicarboxylate was 12 mole % and the yield of dimethyl tricyclodecanedicarboxylate was 81 mole %. The dimethyl tricyclodecanedicarboxylate thus obtained was a transparent, colourless liquid having a viscosity of 65 centipoises at 20°C and a diffraction $n_D^{20}$ of 1.497.

## Example 5
### Preparation of dicarboxylate

Into a 5 litre autoclave were charged 3.0 moles of cobalt oxide and 3.0 moles of pyridine. Hydrogen was then introduced into the autoclave to a pressure of 19.6 bar.G (20 kg/cm².G), after which carbon monoxide was introduced to a pressure of 78.4 bars.G (80 kg/cm².G). The mixture was then reacted at 160°C for 2 hours to synthesize a cobalt carbonyl.

After cooling the reaction mixture, the hydrogen and carbon monoxide were removed, and 4.0 moles of pyridine, 6.0 moles dicyclopentadiene and 15 moles of methanol were charged into the autoclave. Carbon monoxide was then introduced into the autoclave to a pressure of 49.0 bar.G (50 kg/cm².G) and the first hydroesterification was carried out at 110°C for 3 hours and the second hydroesterification was carried out at 140°C for 3 hours in the same way as in Example 4.

From the results of the distillation, it was found that the conversion of dicyclopentadiene was 100 mole %, the yield of methyl tricyclodecenemonocarboxylate was 14 mole % and the yield of dimethyl tricyclodecanedicarboxylate was 78 mole %.

## Example 6

The procedure described in Example 4 was repeated, except that the first hydroesterification reaction was carried out at a temperature of 120°C and the second hydroesterification reaction was carried out at a temperature of 150°C, each for a period of 2 hours.

From the results of the distillation, it was found that the conversion of dicyclopentadiene was 100 mole %, the yield of methyl tricyclodecenemonocarboxylate was 10 mole % and the yield of dimethyltricyclodecanedicarboxylate was 80 mole %.

## Example 7

The procedure described in Example 4 was repeated, except that 800 ml of cyclohexane were used as a reacton solvent.

From the results of the distillation, it was found that the conversion of dicyclopentadiene was 100 mole %, the yield of methyl tricyclodecenemonocarboxylate was 8 mole % and the yield of dimethyl tricyclodecanedicarboxylate was 86 mole %.

## Example 8
### Preparation of monocarboxylate and dicarboxylate in separate stages

(a) Into a 5 litre autoclave were charged 6 moles of dicyclopentadiene, 9 moles of methanol, 7.5 moles of pyridine and 1.5 moles of dicobalt octacarbonyl. Carbon monoxide was then introduced into the autoclave to a pressure of 49.0 bar.G (50 kg/cm$^2$.G), and the reaction mixture was heated to 110°C, at which temperature the reaction was effected for 3 hours. The carbon monoxide pressure was maintained at 29.4 bar.G (30 kg/cm$^2$.G) throughout the reaction by supplying carbon monoxide in an amount corresponding to that consumed by the reaction. After cooling the reaction mixture, the carbon monoxide was removed and the reaction mixture was charged into 5 litres of cyclohexane. The resulting mixture was stirred and then allowed to stand to separate it into the reaction product and the catalyst. The reaction product was distilled under reduced pressure, giving 127 g of a fraction boiling at 30—40°C/0.0013 bar (1 mm Hg) (dicyclopentadiene), 683 g of a fraction boiling at 80—90°C/0.0013 bar (1 mm Hg) (methyl tricyclodecenemonocarboxylate) and 38 g of a fraction boiling at 135—140°C/0.0013 bar (1 mm Hg (dimethyl tricyclodecanedicarboxylate).

(b) Into the same autoclave as was used in step (a) were charged 683 g (3.5 moles) of the methyl tricyclodecenemonocarboxylate obtained in step (a), 6 moles of methanol and 70% of the catalyst separated in step (a) (corresponding to 2.1 moles of cobalt and 4.7 moles of pyridine). Carbon monoxide was then introduced to a pressure of 49.0 bar.G (50 kg/cm$^2$.G). Reaction was then carried out at 140°C for 3 hours. After cooling the reaction mixture, carbon monoxide was removed and the reaction mixture was treated with 3N aqueous hydrochloric acid to decompose the catalyst. The mixture was then washed with water and distilled under reduced pressure, to give 124 g of a fraction boiling at 80—90°C/0.0013 bar (1 mm Hg) (methyl tricyclodecenemonocarboxylate) and 899 g of a fraction boiling at 135—140°C/0.0013 bar (1 mm Hg) (dimethyl tricyclodecanedicarboxylate).

From the results of this distillation, it was found that the conversion of dicyclopentadiene was 84 mole %, the yield of methyl tricyclodecenemonocarboxylate was 11 mole % and the yield of dimethyl tricyclodecanedicarboxylate was 68 mole %.

### Example 9

The procedure described in Example 4 was repeated, except that the reaction mixture produced was fed to 5 litres of cyclohexane under a nitrogen atmosphere and the resulting mixture was stirred and then allowed to stand, upon which the catalyst separated in the lower layer. The amount of cobalt contained in the catalyst thus separated was 99.8% of the amount of cobalt originally used and the amount of pyridine was 89.2% of that originally used.

To the separated catalyst were added 6 moles of dicyclopentadiene, 15 moles of methanol and 0.8 mole of pyridine, and then the mixture was allowed to react in a manner similar to that of Example 4.

From the results of the distillation, it was found that the conversion of dicyclopentadiene was 100 mole %, the yield of methyl tricyclodecenemonocarboxylate was 13 mole % and the yield of dimethyl tricyclodecanedicarboxylate was 78 mole %.

### Example 10
Preparation of dicarboxylate

Into a 5 litre autoclave were charged 2 moles of dicyclopentadiene, 5 moles of methanol, 2.5 moles of pyridine and 0.5 mole of dicobalt octacarbonyl. Carbon monoxide was then introduced into the autoclave to a pressure of 49.0 bar.G (50 kg/cm$^2$.G), after which the contents were heated to 110°C to effect the reaction. The carbon monoxide pressure was maintained at 29.4 bar.G (30 kg/cm$^2$.G) throughout the reaction by supplying carbon monoxide in an amount corresponding to that consumed. The reaction was carried out under these conditions for 3 hours until the hydroesterification conversion of the norbornene ring double bond had reached 90%. A further 2 moles of dicyclopentadiene and 5 moles of methanol were then fed into the autoclave under pressure and the reaction was continued under the same reaction conditions for a further 3 hours. At the end of this time, a further 2 moles of dicyclopentadiene and 5 moles of methanol were fed into the autoclave under pressure and the reaction was continued under the same conditions for a further 3 hours. Finally, the reaction temperature was raised to 140°C and the reaction carried out for a further 3 hours.

After cooling the reaction mixture, the carbon monoxide was removed and the mixture was treated with 3N aqueous hydrochloric acid to decompose the catalyst. It was then washed with water and distilled under reduced pressure.

From the results of the distillation, it was found that the conversion of dicyclopentadiene was 100 mole %, the yield of methyl tricyclodecenemonocarboxylate was 8 mole % and the yield of dimethyl tricyclodecanedicarboxylate was 80 mole %.

### Example 11

The procedure described in Example 4 was repeated, except that, in the second hydroesterification reaction, the reaction temperature was 125°C instead of 140°C and the reaction time was prolonged from 3 hours to 6 hours.

From the results of the distillation, it was found that the conversion of dicyclopentadiene was 100 mole %, the yield of methyl tricyclodecenemonocarboxylate was 16 mole % and the yield of dimethyl tricyclodecanedicarboxylate was 77 mole %.

## Comparative Example 3

The procedure described in Example 4 was repeated, except that the reaction temperature was maintained at 140°C throughout both the first and the second hydroesterification reactions, and the reactions were effected for a total of 4 hours.

From the results of the distillation, it was found that the conversion of dicyclopentadiene was 100 mole %, the yield of methyl tricyclodecenemonocarboxylate was 12 mole % and the yield of dimethyl tricyclodecanedicarboxylate was 60 mole %.

## Comparative Example 4

The procedure described in Example 4 was repeated, except that the amount of pyridine was reduced from 7.5 moles to 2.5 moles and the amount of dicobalt octacarbonyl was reduced from 1.5 moles to 0.5 mole.

From the results of the distillation, it was found that the conversion of dicyclopentadiene was 100 mole %, the yield of methyl tricyclodecenemonocarboxylate was 6 mole % and the yield of dimethyl tricyclodecanedicarboxylate was 54 mole %.

In Examples 4 to 11, a monocarboxylate of the dicyclopentadiene, which is an intermediate product in the preparation of the dicarboxylate, was partially formed. Since this intermediate can easily be separated from the dicarboxylate by distillation, the yield of dicarboxylate can be further increased by returning the intermediate to the reaction system after separation.

## Claims

1. A process for preparing a monocarboxylate ester of a dicyclopentadiene by reacting the dicyclopentadiene with carbon monoxide and an alcohol in the presence of cobalt catalysts, characterized in that the hydroesterification reaction is effected at a temperature of 130°C or less in the presence of at least 0.5 mole (per mole of the dicyclopentadiene) of a pyridine base (which is pyridine or a fused ring derivative of pyridine, and which is substituted or unsubstituted) to hydroesterify the double bond of the norbornene ring.

2. A process according to Claim 1, effected at a carbon monoxide pressure of at least 19.6 bar.G (20 kg/cm².G).

3. A process according to Claim 1 or Claim 2, effected at a temperature of from 80 to 130°C.

4. A process according to Claim 3, in which said temperature is from 100 to 120°C.

5. A process according to any one of the preceding Claims, in which the alcohol is employed in an amount of from 1.0 to 3.0 moles per mole of the dicyclopentadiene.

6. A process for preparing a dicarboxylate ester of a dicyclopentadiene by reacting the dicyclopentadiene with carbon monoxide and an alcohol in the presence of a cobalt catalyst, characterized in that a first hydroesterification reaction is effected at a temperature of 130°C or less in the presence of at least 0.5 mole (per mole of the dicyclopentadiene) of a pyridine base (which is pyridine or a fused ring derivative of pyridine, and which is substituted or unsubstituted) to hydroesterify the double bond of the norbornene ring, thus giving the monocarboxylate ester, and subsequently the remaining double bond of said ester is hydroesterified in a second hydroesterification reaction to give the dicarboxylate of the dicyclopentadiene.

7. A process according to Claim 6, in which the second hydroesterification reaction is effected at a temperature of from 100 to 160°C.

8. A process for preparing a dicarboxylate ester of a dicyclopentadiene by reacting the dicyclopentadiene with carbon monoxide and an alcohol in the presence of a cobalt catalyst, characterized in that the hydroesterification reaction is first carried out at a temperature of 130°C or less in the presence of at least 0.5 mole (per mole of the dicyclopentadiene) of a pyridine base (which is pyridine or a fused ring derivative of pyridine, and which is substituted or unsubstituted), and the temperature is subsequently elevated to a value greater than 130°C to allow the reaction to proceed further.

9. A process according to Claim 8, in which the first esterification reaction is carried out until 80% or more of the norbornene ring double bonds are hydroesterified.

10. A process according to any one of Claims 6 to 9, in which the first hydroesterification reaction is effected at a carbon monoxide pressure of at least 19.6 bar.G (20 kg/cm².G).

11. A process according to any one of Claims 6 to 10, in which the first hydroesterification reaction is effected at a temperature of from 80 to 130°C.

12. A process according to Claim 11, in which said temperature is from 100 to 120°C.

13. A process according to any one of Claims 6 to 12, in which the second hydroesterification reaction is effected at a temperature of from 130 to 150°C.

14. A process according to any one of Claims 6 to 13, in which the alcohol is employed in an amount of from 1.5 to 5.0 moles per mole of the dicyclopentadiene.

15. A process according to any one of the preceding Claims, in which said pyridine base is pyridine itself.

16. A process according to any one of Claims 1 to 14, in which said pyridine base is a methyl derivative, ethyl derivative, dimethyl derivative, trimethyl derivative or methylethyl derivative of pyridine.

17. A process according to Claim 16, in which said pyridine base is $\beta$-picoline, $\gamma$-picoline, 4-ethylpyridine, 3,4-lutidine or $\beta$-collidine.

18. A process according to any one of the preceding Claims, in which said pyridine base is employed in an amount of at least 0.8 mole per mole of the dicyclopentadiene.

19. A process according to any one of the preceding Claims, in which the cobalt catalyst is cobalt carbonyl.

20. A process according to any one of Claims 1 to 18, in which said cobalt catalyst is cobalt oxide, cobalt carbonate, cobalt hydroxide, metallic cobalt, cobalt acetate, cobalt naphthenate or cobalt octenate.

21. A process as claimed in any one of the preceding Claims, in which from 1.5 to 4.0 moles of said pyridine base are employed per gram atom of cobalt in said cobalt catalyst.

22. A process as claimed in any one of the preceding Claims, in which said alcohol is methanol, ethanol, propanol, ethylene glycol, propylene glycol, glycerin or pentaerythritol.

**Patentansprüche**

1. Verfahren zur Herstellung von Monocarbonsäureestern von Dicyclopentadienen durch Umsetzung des Dicyclopentadiens mit Kohlenmonoxid und einem Alkohol in Gegenwart eines Cobaltkatalysators, dadurch gekennzeichnet, daß die Hydroveresterung bei einer Temperatur von 130°C oder darunter in Gegenwart von mindestens 0,5 mol (pro Mol Dicyclopentadien) einer Pyridinbase (die Pyridin oder ein substituiertes oder unsubstituiertes Pyridinderivat mit einem ankondensierten Ring ist) zur Hydroveresterung der Doppelbindung des Norbornenrings durchgeführt wird.

2. Verfahren nach Anspruch 1, dadurch gekennzeichnet, daß bei einem Kohlenmonoxidüberdruck von mindestens 19,6 bar (20 kg/cm²) umgesetzt wird.

3. Verfahren nach Anspruch 1 oder 2, dadurch gekennzeichnet, daß bei einer Temperatur von 80 bis 130°C umgesetzt wird.

4. Verfahren nach Anspruch 3, dadurch gekennzeichnet, daß bei einer Temperatur von 100 bis 120°C umgesetzt wird.

5. Verfahren nach einem der Ansprüche 1 bis 4, dadurch gekennzeichnet, daß der Alkohol in einer Menge von 1,0 bis 3,0 mol pro Mol Dicycloentadien eingesetzt wird.

6. Verfahren zur Herstellung von Dicarbonsäureestern von Dicyclopentadienen durch Umsetzung des Dicyclopentadiens mit Kohlenmonoxid une einem Alkohol in Gegenwart eines Cobaltkatalysators, gekennzeichnet durch eine erste Hydroveresterung bei einer Temperatur von 130°C oder darunter in Gegenwart von mindestens 0,5 mol (pro Mol Dicyclopentadien) einer Pyridinbase (die Pyridin oder ein substituiertes oder unsubstituiertes Pyridinderivat mit ankondensiertem Ring ist) zur Hydroverestung der Doppelbindung des Norbornenrings unter Erhalt des Monocarbonsäureesters und anschließende zweite Hydroveresterung der verbliebenen Doppelbindung des Esters zum Dicarbonsäureester des Dicyclopentadiens.

7. Verfahren nach Anspruch 6, dadurch gekennzeichnet, daß die zweite Hydroveresterung bei einer Temperatur von 100 bis 160°C durchgeführt wird.

8. Verfahren zur Herstellung von Dicarbonsäureestern von Dicyclopentadienen durch Umsetzung des Dicyclopentadiens mit Kohlenmonoxid und einem Alkohol in Gegenwart eines Cobaltkatalysators, dadurch gekennzeichnet, daß die Hydroveresterung zuerst bei einer Temperatur von 130°C oder darunter in Gegenwart von mindestens 0,5 mol (pro Mol Dicyclopentadien) einer Pyridinbase (die Pyridin oder ein substituiertes oder unsubstituiertes Pyridinderivat mit ankondensiertem Ring ist) durchgeführt und die Temperatur anschließend zur weiteren Umsetzung auf einen Wert über 130°C erhöht wird.

9. Verfahren nach Anspruch 8, dadurch gekennzeichnet, daß die erste Hydroveresterung so lange durchgeführt wird, bis 80% oder mehr der Doppelbindungen des Norbornenrings hydrover-estert sind.

10. Verfahren nach einem der Ansprüche 6 bis 9, dadurch gekennzeichnet, daß die erste Hydroveresterung bie einem Kohlenmonoxidüberdruck von mindestens 19,6 bar (20 kg/cm²) durchgeführt wird.

11. Verfahren nach einem der Ansprüche 6 bis 10, dadurch gekennzeichnet, daß die erste Hydroveresterung bei einer Temperatur von 80 bis 130°C durchgeführt wird.

12. Verfahren nach Anspruch 11, dadurch gekennzeichnet, daß die erste Hydroveresterung bei einer Temperatur von 100 bis 120°C durchgeführt wird.

13. Verfahren nach einem der Ansprüche 6 bis 12, dadurch gekennzeichnet, daß die zweite Hydroveresterung bei einer Temperatur von 130 bis 150°C durchgeführt wird.

14. Verfahren nach einem der Ansprüche 6 bis 13, dadurch gekennzeichnet, daß der Alkohol in einer Menge von 1,5 bis 5,0 mol pro Mol Dicyclopentadien eingesetzt wird.

15. Verfahren nach einem der Ansprüche 1 bis 14, dadurch gekennzeichnet, daß als Pyridinbase Pyridin verwendet wird.

16. Verfahren nach einem der Ansprüche 1 bis 14, dadurch gekennzeichnet, daß als Pyridinbase ein Methyl-, Ethyl-, Dimethyl-, Trimethyl- oder Methylethylpyridinderivat verwendet wird.

17. Verfahren nach Anspruch 16, dadurch gekennzeichnet, daß als Pyridinbase $\beta$-Picolin, $\gamma$-Picolin, 4-Ethylpyridin, 3,4-Lutidin oder $\beta$-Collidin verwendet werden.

18. Verfahren nach einem der Ansprüche 1 bis 17, dadurch gekennzeichnet, daß die Pyridinbase in einer Menge von mindestens 0,8 mol pro Mol Dicyclopentadien eingesetzt wird.

19. Verfahren nach einem der vorhergehenden Ansprüche, dadurch gekennzeichnet, daß als Cobaltkatalysator Cobaltcarbonyl verwendet wird.

20. Verfahren nach einem der Ansprüche 1 bis 18, dadurch gekennzeichnet, daß als Cobaltkatalysator Cobaltoxid, Cobaltcarbonat, Cobalthydroxid, Cobaltacetat, Cobaltnaphthenat oder Cobalt-octenat oder metallisches Cobalt verwendet werden.

21. Verfahren nach einem der Ansprüche 1 bis 20, dadurch gekennzeichnet, daß 1,5 bis 4,0 mol Pyridinbase pro g-Atom Cobalt im Cobaltkatalysator verwendet werden.

22. Verfahren nach einem der Ansprüche 1 bis 21, dadurch gekennzeichnet, daß als Alkohol, Methanol, Ethanol, Propanol, Ethylglycol, Propylenglycol, Glycerin oder Pentaerythrit verwendet werden.

## Revendications

1. Procédé pour la préparation d'un ester monocarboxylate d'un dicyclopentadiène par la mise en réaction du dicyclopentadiène avec l'oxyde de carbone et un alcool en présence d'un catalyseur au cobalt, caractérisé en ce que la réaction d'hydroestérification est effectuée à une température de 130°C ou moins, en présence d'au moins 0,5 mol (par mol du dicyclopentadiène) d'une base pyridinique (qui est la pyridine ou un dérivé à noyaux condensés de la pyridine et qui est substituée ou non substituée) afin d'hydroestérifier la double liaison du noyau norbornène.

2. Procédé selon la revendication 1, caractérisé en ce qu'il est mené sous une pression d'oxyde de carbone d'au moins 19,6 bar.G (20 kg/cm²).

3. Procédé selon la revendication 1 ou 2, caractérisé en ce qu'il est mené à une température de 80 à 130°C.

4. Procédé selon la revendication 1 ou 2, caractérisé en ce qu'il est mené à une température de 100 à 120°C.

5. Procédé selon l'une quelconque des revendications 1 à 4, caractérisé en ce que l'alcool est utilisé dans une proportion de 1,0 à 3,0 mol par mol du dicyclopentadiène.

6. Procédé pour la préparation d'un ester dicarboxylate d'un dicyclopentadiène par la mise en réaction du dicyclopentadiène avec l'oxyde de carbone et un alcool en présence d'un catalyseur au cobalt, caractérisé en ce qu'une première réaction d'hydroestérification est effectuée à une température de 130'C ou moins, en présence d'au moins 0,5 mol (par mol du dicyclopentadiène) d'une base pyridinique (qui est la pyridine ou un dérivé à noyaux condensés de la pyridine et qui est substituée ou non substituée) afin d'hydroestérifier la double liaison du noyau norbornène, ce qui donne l'ester monocarboxylate, puis la double liaison restante de cet ester est hydroestérifiée en une seconde réaction d'hydroestérification pour donner le dicarboxylate du dicyclopentadiène.

7. Procédé selon la revendication 6, caractérisé en ce que la seconde réaction d'hydroestérification est effectuée à une température de 100 à 160°C.

8. Procédé pour la préparation d'un ester dicarboxylate d'un dicyclopentadiène par la mise en réaction du dicyclopentadiène avec l'oxyde de carbone et un alcool en présence d'un catalyseur au cobalt, caractérisé en ce que la réaction d'hydroestérification est tout d'abord menée à une température de 130°C ou moins, en présence d'au moins 0,5 mol (par mol du dicyclopentadiène) d'une base pyridinique (qui est la pyridine ou un dérivé à noyaux condensés de la pyridine et qui est substituée ou non substituée), puis la température est élevée à une valeur supérieure à 130'C afin que la réaction se poursuive.

9. Procédé selon la revendication 8, caractérisé en ce que la première réaction d'estérification est effectuée jusqu'à ce que 80% ou plus des doubles liaisons du noyau norbornène soient hydroestérifiées.

10. Procédé selon l'une quelconque des revendications 6 à 9, caractérisé en ce que la première réaction d'hydroestérification est effectuée sous une pression d'oxyde de carbone d'au moins 19,6 bar.G (20 kg/cm².G).

11. Procédé selon l'une quelconque des revendications 6 à 10, caractérisé en ce que la première réaction d'hydroestérification est effectuée à une température de 80 à 130°C.

12. Procédé selon la revendication 11, caractérisé en ce que la température est comprise entre 100 et 120°C.

13. Procédé selon l'une quelconque des revendications 6 à 12, caractérisé en ce que la seconde réaction d'hydroestérification est menée à une température de 130 à 150°C.

14. Procédé selon l'une quelconque des revendications 6 à 13, caractérisé en ce que l'alcool est utilisé dans une proportion de 1,5 à 5,0 mol par mol de dicyclopentadiène.

15. Procédé selon l'une quelconque des revendications 1 à 14, caractérisé en ce que la base pyridinique est la pyridine elle-même.

16. Procédé selon l'une quelconque des revendications 1 à 14, caractérisé en ce que la base pyridinique est un dérivé méthylique, un dérivé éthylique, un dérivé diméthylique, un dérivé triméthylique ou un dérivé méthyléthylique de la pyridine.

17. Procédé selon la revendication 16, caractérisé en ce que la base pyridinique est la β-picoline, la γ-picoline, la 4-éthylpyridine, la 3,4-lutidine ou la β-collidine.

18. Procédé selon l'une quelconque des revendications 1 à 17 caractérisé en ce que la base pyridinique est utilisée dans une proportion d'au moins 0,8 mol par mol du dicyclopentadiène.

19. Procédé selon l'une quelconque des revendications 1 à 18, caractérisé en ce que la catalyseur au cobalt est un cobalt-carbonyle.

20. Procédé selon l'une quelconque des revendications 1 à 18, caractérisé en ce que le catalyseur au cobalt est l'oxyde de cobalt, le carbonate de cobalt, l'hydroxyde de cobalt, le cobalt métallique, l'acétate de cobalt, le naphténate de cobalt ou l'octénate de cobalt.

21. Procédé selon l'une quelconque des revendications 1 à 20, caractérisé en ce qu'on utilise de 1,5 á 4,0 mol de la base pyridinique par atome-gramme de cobalt dans le catalyseur au cobalt.

22. Procédé selon l'une quelconque des revendications 1 à 21, caractérisé en ce que l'alcool est le méthanol, l'éthanol, le propanol, l'éthylène-glycol, la glycérine ou le penta-érythrol.